(19) Europäisches Patentamt
European Patent Office
Office européen des brevets

(11) EP 1 948 274 B1

(12) **EUROPÄISCHE PATENTSCHRIFT**

(45) Veröffentlichungstag und Bekanntmachung des Hinweises auf die Patenterteilung:
**11.01.2017 Patentblatt 2017/02**

(21) Anmeldenummer: **06819402.6**

(22) Anmeldetag: **10.11.2006**

(51) Int Cl.:
***A61M 15/00*** (2006.01) ***B21G 1/00*** (2006.01)

(86) Internationale Anmeldenummer:
**PCT/EP2006/068356**

(87) Internationale Veröffentlichungsnummer:
**WO 2007/057362 (24.05.2007 Gazette 2007/21)**

(54) **NADEL ZUM LOCHEN VON PULVERKAPSELN FÜR DIE INHALATION**

NEEDLE FOR PUNCTURING POWDER CAPSULES FOR INHALATION

AIGUILLE POUR PERCER DES CAPSULES DE POUDRE POUR L'INHALATION

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **15.11.2005 DE 102005054383**

(43) Veröffentlichungstag der Anmeldung:
**30.07.2008 Patentblatt 2008/31**

(73) Patentinhaber: **Boehringer Ingelheim Pharma GmbH & Co. KG**
**55216 Ingelheim am Rhein (DE)**

(72) Erfinder:
- **HOELZ, Hubert**
  **55413 Oberheimbach (DE)**
- **LUSTENBERGER, Stefan**
  **55457 Gensingen (DE)**
- **KUEHN, Torsten**
  **55437 Appenheim (DE)**
- **WACHTEL, Herbert**
  **55218 Ingelheim Am Rhein (DE)**
- **KUHN, Rolf**
  **55218 Ingelheim Am Rhein (DE)**

(74) Vertreter: **Simon, Elke Anna Maria et al**
**Boehringer Ingelheim GmbH**
**Binger Strasse 173**
**55216 Ingelheim am Rhein (DE)**

(56) Entgegenhaltungen:
**EP-A- 0 582 276**
**EP-A- 0 911 047**
**WO-A-2004/082750**
**DE-U1-202004 005 618**
**US-A- 5 601 475**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft einen Pulverinhalator mit mindestens einer speziell geschliffenen Nadel, um ein präzises Lochen oder Aufschneiden von Kapseln und damit eine optimierte Ausbringung von pulverförmigen Arzneimitteln, Arzneimittelformulierungen oder Arzneimittelmischungen zu gewährleisten.

Pulverinhalatoren sind aus dem Stand der Technik bekannt. Beispielsweise aus EP 0 703 800 B1 oder der EP 0 911 047 A1, welche einen Pulverinhalator, bestehend aus einem schalenförmigen Unterteil und einem ebenfalls schalenförmigen Deckel, offenbaren. Der Patient kann nach dem Einlegen der Kapsel in die Kapselhalterung ein Betätigungsorgan drücken, welches aus einer Ruhestellung in eine Bewegung versetzbar ist und dabei mit mindestens einer in die Kapselhalterung einstoßbaren Nadel zusammenwirkt. Mit Hilfe der Nadel bzw. der Nadeln wird die Kapsel angestochen und das Arzneimittel freigesetzt.

Die WO 2004/082750 A1 zeigt ein System zur Inhalation von trockenem Pulver, wobei sich ein aktives, mikronisiertes Ingredienz in einer länglichen HPMC-Kapsel befindet. Das Inhalationsgerät ist mit beispielsweise zwei angeschliffenen Anstechnadeln von Durchmessern im Bereich von 0,8 mm bis 3 mm ausgestattet, mittels derer die Kapsel an ihren Enden angestochen werden kann. Inhaliert ein Patient am Mundstück des Geräts, kann die Kapsel im Innern es Geräts rotieren und das Pulver kann durch die zuvor gestochenen Löcher austreten und über das Mundstück in die Atemwege des Patienten gelangen.

Weitere Inhalatoren sind unter den Markennamen Spinhaler®, Rotahaler®, Aerolizer®, Flowcaps®, Turbospin®, AIR DPI®, Orbital®, Directhaler® bekannt und/oder in DE 33 45 722, EP 0 591 136, DE 43 18 455, WO 91/02558, FR-A-2 146 202, US-A-4 069 819. EP 666085, EP 869079, US3991761, WO99/45987, WO 200051672, Bell, J. Pharm. Sci. 60, 1559 (1971); Cox, Brit. Med. J. 2, 634 (1969), beschrieben.

In der Medizin sind Nadeln für die verschiedensten Anwendungsmöglichkeiten bekannt, sei es zur Diagnose (Biopsien), zur Therapie (Injektionen) oder auch zum Lochen oder Aufschneiden von Kapseln, die in Inhalatoren zur Anwendung kommen.

Von besonderer Bedeutung ist beim Lochen oder Aufschneiden der Kapsel - im Gegensatz zur Perforierung von menschlicher oder tierischer Haut - immer die Erzeugung einer präzisen und großen Einstichstelle, um eine möglichst vollständige Ausbringung des Pulvers aus der Kapsel zu erzielen.

Eine präzise Einstichstelle oder Schnittstelle kann nicht von einer stumpfen Nadel erzeugt werden, da diese die Kapseln erfahrungsgemäß lediglich eindrückt und beschädigt. Somit wäre keine vollständige Ausbringung des Pulvers und damit des Arzneimittels, der Arzneimittelformulierung oder Arzneimittelmischung möglich,

Die Nadel muß außerdem einen speziellen Schliff aufweisen, um eine Kapsel präzise zu Lochen oder Aufzuschneiden, um nachfolgend die möglichst vollständige Ausbringung des Pulvers zu gewährleisten.

Die Deutsche Norm DIN 13097 enthält Festlegungen zu Anschliffarten für medizinische Kanülen für die Injektion und Transfusion aus starren und halbstarren Werkstoffen. Diese Anschliffarten sind der Einfach-, Facetten und der Hinterschliff. Weiterhin existiert die europäische Norm EN ISO 7864 für sterile Einmalinjektionskanülen.

Aus der deutschen Gebrauchsmusterschrift DE 20 2004 005 618 sind beispielsweise Spezialnadeln zur Punktion von Medikamentenkapseln für den Einsatz in Pulverinhalatoren bekannt. Hierbei handelt es sich um Vollmaterialnadeln mit einem schrägen Grundschliff und zwei vorderen Facettenschliffen mit speziell definierten Schliffwinkeln.

Die Kapseln hat die Aufgabe, die gesamte Inhalationsformulierung, welche den Wirkstoff enthält, vor chemischen oder physikalichen Veränderungen zu schützen, bevor ein Patient die Formulierung inhaliert.

Die Kapsel besteht in der Regel aus zwei Teilen, einem Kapselkörper (Körper) und einer Kapselkappe (Kappe), die teleskopartig ineinander geschoben werden. Aber auch mehrteilige Kapseln sind bekannt.

Das Kapselmaterial ist eine Gelatine, insbesondere Hartgelatine. Für einige spezielle Anwendungen werden als Kapselmaterial auch für den Menschen gut verdauliche, wasserlösliche Dunststoffe verwendet, um bei oraler Verabreichung den Wirkstoff in bestimmten Kompartimenten des Magen-Darm-Trakts freizusetzen.

Im Folgenden sind Beispiele für verschiedene Kapselmaterialien aufgeführt: EP 0460921 beschreibt Kapseln aus Chitosan und Stärke, Getreidepulver, Oligosacchariden, Methacrylsäure-Methylacrylat, Methacrylsäure-Ethylacrylat, Hydroxypropylmethyl-Celluloseacetat, -succinat oder -phthalat. Das Kapselmaterial zeichnet sich dadurch aus, dass der Inhalt erst im Dickdarm freigesetzt wird

GB 938828 offenbart Kapseln für radioaktive Substanzen im therapeutischen oder diagnostischen Einsatz. Die Kapseln bestehen aus wasserlöslicher Gelatine, Methylcellulose, Polyvinylalkohol oder wasserlöslichen nichttoxischen Thermoplasten.

WO 00/07572 offenbart Kapseln für Inhalatoren, welche aus nicht-verdaulichem Kunststoff bestehen,

Aufgabe der Erfindung ist die Bereitstellung eines Pulverinhalators mit mindestens einer Nadel, welche einen speziellen Schliff für das präzise Lochen oder Aufschneiden einer Kapsel besitzt, um eine definierte Menge eines Arzneimittels, einer Arzneimittelformulierung oder -mischung aus den in Pulverinhalatoren verwendeten Kapseln auszubringen.

Die obige Aufgabe wird durch einen Pulverinhalator gemäß Anspruch 5 gelöst. Vorteilhafte Weiterbildungen

sind Gegenstand der Unteransprüche.

Aufgabe der Erfindung ist weiterhin die Bereitstellung einer Nadel, welche durch ihre spezielle Schliffform eine verbesserte Ausbringung von Arzneimitteln, Arzneimittelformulierungen oder Arzneimittelmischungen gewährleistet, indem die Kapsel im Pulverinhalator präzise gelocht oder aufgeschnitten wird, wobei für den Patienten einen reduzierter Arbeitsaufwand erforderlich ist.

Die Aufgabe wird durch eine Nadel gemäß Anspruch 1 gelöst. Vorteilhafte Weiterbildungen sind Gegenstand der Unteransprüche.

Wie schon oben ausgeführt, sind zahlreiche Nadeln und Schliffarten aus dem Stand der Technik bekannt.

Eine Rahmenbedingung, die der Übertragung des Standes der Technik auf die vorliegende Aufgabe entgegensteht, ist die Befüllung der Kapsel mit einer pharmazeutischen Formulierung.

Eine weitere Rahmenbedingung stellt die Abmessung und Wandstärke der zu lochenden oder aufzuschneidenden Kapseln dar und hier insbesondere die dünne Wandung einer solchen Kapsel. Diese ist notwendig, da die Kapsel in einem handelsüblichen Inhalator verwendet werden können soll. Dort muss sie in einfacher Art und Weise ohne großen Arbeitsaufwand für den Patienten aufgestochen oder aufgeschnitten werden können.

Die vorliegende Erfindung betrifft vorzugsweise ein Ensemble aus einem Inhalator für die Inhalation pulverförmiger Arzneimittel und einer zweiteiligen Kapsel, wobei der Inhalator gekennzeichnet ist durch a) ein nach oben hin offenes, becherförmiges Unterteil (1), welches in der Ummantelung zwei gegenüber liegende Fenster (2) aufweist und am Rand der Öffnung ein erstes Scharnierelement mit einem Gelenkbolzen (3) hat, b) eine Platte (9), welches die Öffnung des Unterteils (1) bedeckt und ein zweites Scharnierelement aufweist, sowie eine Siebhalterung (11) mit einem Sieb (10) trägt, c) eine versenkbare Kapselhalterung (4) zum Aufnehmen der Kapsel, die senkrecht zur Plattenebene an der zum Unterteil weisenden Seite der Platte (9) ausgebildet ist und an der ein gegen eine Feder beweglicher Kopf vorgesehen ist, wobei der Kopf mit ein oder zwei geschliffenen Nadeln (6) versehen ist, d) ein Mundstück (12) mit einem Mundrohr und ggfls. einer Griffhilfe (17) und einem dritten Scharnierelement, sowie e) einen Deckel (13), der ein viertes Scharnierelement aufweist, wobei die Scharnierelemente eins des Unterteils, zwei der Platte, drei des Oberteils und vier des Deckels miteinander verbunden sind. Außerdem besitzt der Inhalator ein Betätigungsorgan (7), welches zum Öffnen des Deckels (13) dient, indem das Verschlusselement (14) am Deckel (13) auf die geneigte Seitenwand (15) (gglfs. mit eine Riffelung (16)) der Ausnehmung (8) stösst, welche bei weiterem Vorschub des Betätigungsorgans (7) als Gleitfläche wirkt und für ein Lösen des Deckels (13) sorgt.

Die Führung der Nadel bzw. der Nadeln wird im wesentlichen über zwei seitlich angeordnete Führungsarme (18) realisiert. Die Führungsarme haben außerdem die Aufgabe, das Betätigungsorgan (7) unter einer Vorspannung zu halten. Hierfür sind die Führungsarme (18) an ihrem hauptkörperfernen Ende mit Endanschlägen versehen, die in der Ruhestellung des Betätigungsorgans (7) an den Führungshülsen der Kapselhalterung (4) anliegen. Die Führungshülsen befinden sich an der Außenseite der Kapselhalterung (4). Zwischen den Führungsarmen (18) ist eine Schraubenfeder (5) angeordnet, die in ihrer axialen Erstreckung parallel zu der bzw. den Nadeln (6) verläuft, wobei die Schraubenfeder (5) derart mit der Länge der Führungsarme (18) abgestimmt ist, dass das Betätigungsorgan (7) auch in Ruhestellung vorgespannt ist. Ein derartiger Inhalator ist in Figur 1 gezeigt.

Die Kapsel wird mit einem pulverförmigen, inhalierbaren Arzneimittel, Arzneimittelformulierung oder Arzneimittelgemisch, gegebenenfalls noch zusätzlich ein Trägermaterial enthaltend, befüllt.

Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:

- **W** stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar, kombiniert mit einem Betamimetika, Corticosteroiden, PDE4-Inhibitoren, EGFR-Hemmern oder LTD4-Antagonisten,
- **W** stellt ein Corticosteroiden dar, kombiniert mit einem PDE4-Inhibitoren, EGFR-Hemmern oder LTD4-Antagonisten
- **W** stellt ein PDE4-Inhibitoren dar, kombiniert mit einem EGFR-Hemmern oder LTD4-Antagonisten
- **W** stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und

- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1 -benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1 -Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid

gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1**

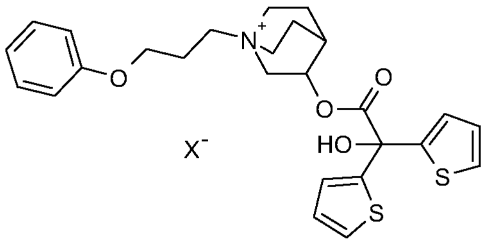

**AC-1**

worin X - ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en**

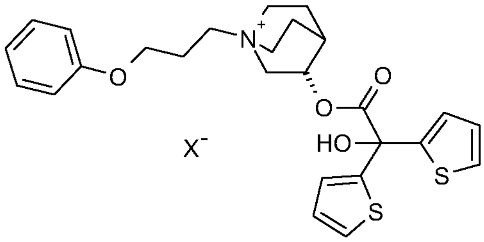

**AC-1-en**

enthalten, worin X - die vorstehend genannten Bedeutungen aufweisen kann. Weiterhin bevorzugte Anticholinergika sind ausgewählt aus den Salzen der Formel **AC-2**

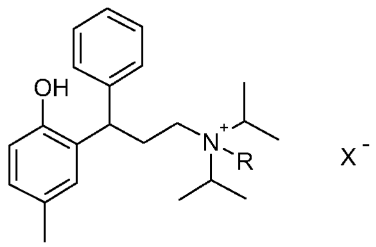

**AC-2**

worin R entweder Methyl oder Ethyl bedeuten und worin X - die vorstehend genannte Bedeutungen aufweisen kann. In einer alternativen Ausführungsform kann die Verbindung der Formel AC-2 auch in Form der freien Base AC-2-base vorliegen.

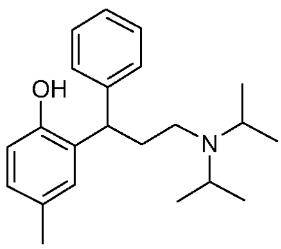

**AC-2-base**

Weiterhin genannte Verbindungen sind:

- 2,2-Diphenylpropionsäuretropenolester-Methobromid
- 2,2-Diphenylpropionsäurescopinester-Methobromid

- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X- genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und

- 6,9-Difluor-17-[(2-furanylcarbonyl)oxy]-11 -hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6$\alpha$,9$\alpha$-difluoro-11$\beta$-hydroxy-16a-methyl-3-oxo-17a-(2,2,3,3-tertamethylcyclopropylcarbonyl)oxy-androsta-1,4-diene-17$\beta$-carbonsäure cyanomethyl ester

gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, CI-1018, CDC-801, CDC-3052, D-22888, YM-58997, Z-15370 und

- N-(3,5-Dichloro-1-oxo-pyridin-4-yl)-4-difluormethoxy-3-cyclopropylmethoxybenzamid
- (-)p-[(4*a*R*,10*b*S*)-9-Ethoxy-1,2,3,4,4a, 10b-hexahydro-8-methoxy-2-methylbenzo[s][1,6]naphthyridin-6-yl]-N, N-diisopropylbenzamid

- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxyphenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9H-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin

gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und

- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und

- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]-amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin

- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinyl-carbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yl-oxy}-7-methoxy-chinazolin
- 4-[(3-Chlor-4-fluor-phenyl)amino]-6-{1-[(methoxymethyl)carbonyl]-piperidin-4-yl-oxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin

- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy- chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxyethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin

gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

Die Nadel kann aus allen, dem Fachmann bekannten Materialien bestehen. Insbesondere kann sie aus folgenden Materialien bestehen:

Schlagzäher Kunststoff, beispielsweise POM (Polyoxymethylen bzw. Polyformaldehyd), ABS (Acrylnitril-Butadien-Styrol-Copolymere), PEEK (Polyetheretherketon), pharmazeutisch unbedenklicher Edelstahl beispielsweise der Zusammensetzung X5CrNi18-10, Werkstoffnummer 1.4301. Alternativen sind andere A2 Cr-Ni Stähle der V2A-Reihe, z. B. Werkstoffnummer 1.4567 oder 1.4541, sowie A4 Stähle Cr-Ni-Mo der V4A-Reihe, beispielsweise Werkstoffnummer 1.4404, 1.4401 oder 1.4435.

Es kann sich hierbei um eine Vollmaterialnadel oder um eine Hohlnadel (Kanüle) handeln.

Der Nadelschliff der Nadel bzw. Nadeln im erfindungsgemäßen Inhalator an einer Vollmaterialnadel oder an einer Hohlnadel kann ein sogenannter Dreikantschliff sein, wie in anliegender Zeichnung offenbart.

Der Nadelschliff der Nadel bzw. Nadeln im erfindungsgemäßen Inhalator an einer Vollmaterialnadel oder an einer Hohlnadel kann ein sogenannter Hinterschliff sein, wie grundsätzlich aus der Deutschen Norm, DIN 13097, für medizinische Kanülen vom September 2002 bekannt.

Der Nadelschliff der Nadel bzw. Nadeln im erfindungsgemäßen Inhalator an einer Hohlnadel kann ein sog. Facettenschliff sein, wie grundsätzlich aus der Deutschen Norm, DIN 13097, für medizinische Kanülen vom September 2002 bekannt.

Für alle Nadeln gilt:

Der Nadeldurchmesser beträgt vorzugsweise 1.00-2.00 mm mit einem ultralangen, langen, mittleren oder kurzen Schliff. Die Schlifflängen sind beispielsweise: Kurzer Schliff = 0.5 - 3 mm; Mittlerer Schliff = 4 - 7 mm; Langer Schliff = 8 - 11 mm; Ultralanger Schliff = 12 - 15 mm.

Der erfindungsgemäße Inhalator beinhaltet mindestens eine Nadel, vorzugsweise zwei Nadeln, zum Lochen oder Aufschneiden, vorzugsweise zum Lochen, einer in einem Inhalator befindlichen Kapsel zur Inhalation eines Arzneimittels, einer Arzneimittelformulierungen oder Arzneimittelmischung, wobei die beiden Nadeln jeweils als Vollmaterial- oder Hohlmaterialnadeln mit einem Dreikant- oder einem Hinterschliff oder als Hohlnadel mit einem Facettenschliff vorhanden sein kann. Ein Ausführungsbeispiel hierzu ist in Figur 2 dargestellt.

Bevorzugt wird die Verwendung von zwei Dreikantnadeln, welche als Vollmaterialnadeln ausgebildet sind und ganz besonders bevorzugt zum Lochen einer nicht-verdaulichen Kunststoffkapsel eingesetzt werden. Ganz besonders bevorzugt ist hierbei die Verwendung von einer Kombination aus einer Dreikantnadel und einer Hinterschliffnadel.

Die folgenden Beispiele zeigen eine deutliche Korrelation zwischen der Lochgröße in der Kapsel, der Präzision der Einstichstelle und der ausgebrachten Dosis. Der Begriff "ausgebrachte Dosis" wird vom Fachmann auch als "delivered dose" bezeichnet und bezieht sich auf die Dosismenge des ausgestossenen Wirkstoffs, welche im Dosissammelrohr nach Pharmacopeia gemessen wird.

Im Detail erfolgte die Bestimmung der ausgebrachten Dosis durch die Bestimmung der Masse der gefüllten Kapsel und Ausbringung ins Dosisrohr nach Pharm. Eur. 4. Auflage (2002) S. 554 ff. (oder USP für USA). Anschließend wird eine Differenzwägung zur Bestimmung der gefüllten im Vergleich zur ausgebrachten Kapselmasse durchgeführt.

Im Gegensatz zur "Delivered Dose" wird als "Delivered Mass" die Gesamtmenge an Pulver, d.h. incl. Formulierungsmaterial beispielsweise, bezeichnet, die ausgebracht wird. Die Bestimmung der "Delivered Mass" erfolgt durch die Wägung der vollen im Vergleich zur entleerten Kapsel und der Berechnung der Gewichtsdifferenz.

Figur 3 zeigt Löchergeometrien, wie sie unter Verwendung verschiedener Nadeln beobachtet werden.
Die Einstichstelle wurde mit einem Mikroskop der Firma Zeiss, Marke Axioplan untersucht und nachfolgend mit einer Kamera der Firma Olympus, Marke DP10 fotografiert. Die Analyse der Bilder und damit des Lochs erfolgte unter Einsatz der Software der Firma SIS Analysis, Version 3.0.

Figur 4 veranschaulicht die Korrelation zwischen ausgebrachter Dosis und Kapsellochfläche mit Hinterschliffnadeln (Vollmaterialnadeln).
Es wurden zwei unterschiedliche Nadelsorten (Typ 1 und Typ 2) getestet.

Figur 5 veranschaulicht die Korrelation zwischen Kapsel-Lochfläche und ausgebrachter Dosis bei Facettenschliffnadeln (Vollmaterialnadeln).
Die durchgezogene Linie ist ein Trend, ermittelt durch die Methode der kleinsten Fehlerquadrate.

Vergleich der verschiedenen Schliffarten:

Die Messungen wurden von 4 Geräten mit je 10 Kapseln erhoben, nachdem jeweils in einem ersten Übungslauf in je einem Gerät 10 Kapseln entleert wurden. Die Delivered Mass (%) ist das prozentuale Verhältnis der Massendifferenz (volle Kapsel - entleerte Kapsel)/Füllmasse.

Formel:

$$\text{Delivered Mass (\%)} = 100 * (m(\text{voll}) - m(\text{leer})) / m(\text{Füllung})$$

| Vollmaterialnadel | Anzahl Messungen | Mittelwert Delivered Mass (%) | Varianz (%$^2$) | Std.-Abweichung (%) |
|---|---|---|---|---|
| Facette | 40 | 83.1 | 96.0 | 9.8 |
| Standard | 40 | 85.5 | 62.0 | 7.9 |
| Dreikant | 40 | 95.1 | 58.8 | 7.7 |

Vorteil der Dreikantnadel ist eindeutig eine hohe Delivered Mass und eine kleine Standardabweichung.

Die Versuchsergebnisse zeigen eine erhöhte Ausbringung von Kapselmaterial bei einer speziellen Nadelschliffform, welche eine präzise Lochung bzw. ein präzises Aufschneiden einer Kapsel gewährleistet.
Als Kapselfüllmaterial wird in allen Experimenten Lactose verwendet.
Als Kapseln wurden Gelatine- und Kunststoffkapseln getestet.

## Patentansprüche

1. Nadel zum Lochen oder Aufschneiden von Kapseln in einem Pulverinhalator zur Ausbringung von Arzneimitteln, Arzneimittelformulierungen oder Arzneimittelmischungen, **dadurch gekennzeichnet, dass** die Nadel eine Vollmaterialnadel mit einem Dreikant- oder einem Hinterschliff ist, und die Nadel einen Durchmesser von 1.00 - 2.00 mm besitzt, wobei die Nadel eine zentrale Längsachse und eine Nadelspitze aufweist, die durch den Dreikant- oder Hinterschliff gebildet wird und die auf der zentralen Längsachse liegt.

2. Nadel gemäß Anspruch 1, **dadurch gekennzeichnet, dass** die Nadel einen ultralangen, langen, mittleren oder kurzen Schliff besitzt.

3. Nadel gemäß einem der Ansprüche 1 oder 2, **dadurch gekennzeichnet, dass** die Nadel aus schlagzähem Kunststoff oder pharmazeutisch unbedenklichem Edelstahl besteht.

4. Nadel gemäß einem oder mehreren der Ansprüche 1-3, **dadurch gekennzeichnet, dass** die Nadel zum Lochen einer nicht-verdaulichen Kunststoffkapsel im Pulverinhalator dient.

5. Inhalator für die Inhalation pulverförmiger Arzneimittel aus Kapseln, aufweisend

   - ein Unterteil (1)
   - eine mit dem Unterteil (1) verrastbare Platte (9), mit welcher das Unterteil (1) verschließbar ist, und eine in dem Unterteil (1) versenkbare Kapselhalterung (4) zur Aufnahme der Kapseln,
   - ein mit der Platte (9) verrastbares Mundstück (12),
   - einen Deckel (13), welcher das Mundstück (12) in einer Verschlussposition abdeckt und mittels eines Verschlusselementes (14) verrastet,
   wobei das Unterteil (1), die Platte, das Mundstück (12) und der Deckel (13) durch ein einziges Gelenk miteinander gelenkig verbunden sind, und
   - ein Betätigungsorgan (7), welches aus einer Ruhestellung in eine Bewegung versetzbar ist und dabei mit mindestens einer in die Kapselhalterung (4) einstoßbaren Nadel (6) zusammenwirkt,

   **dadurch gekennzeichnet, dass**
   die Nadel (6) gemäß einem der Ansprüche 1-4 ausgebildet ist.

6. Inhalator gemäß Anspruch 5, **dadurch gekennzeichnet, dass** zwei Dreikantnadeln verwendet werden, welche als Vollmaterialnadel ausgebildet sind.

7. Inhalator gemäß Anspruch 6, **dadurch gekennzeichnet, dass** die zwei Dreikantnadeln parallel zueinander in einem gegen eine Feder beweglichen Kopf angeordnet sind.

8. Inhalator gemäß einem oder mehreren der Ansprüche 5-7, **dadurch gekennzeichnet, dass** Nadel bzw. die Nadeln zum Lochen einer Kapsel aus einem nicht-verdaulichen Kunststoff verwendet wird.

## Claims

1. Needle for puncturing or cutting open capsules in a powder inhaler used for dispensing medicines, medicine formulations or medicine mixtures, **characterised in that** the needle is a solid needle comprising a ground portion that is triangular or relief-ground, and the needle has a diameter of from 1.00 to 2.00 mm, the needle having a central longitudinal axis and a needle tip which is formed by the triangular or relief-ground ground portion and which lies on the central longitudinal axis.

2. Needle according to claim 1, **characterised in that** the needle has an ultra-long, long, medium-length or short ground portion.

3. Needle according to either claim 1 or claim 2, **characterised in that** the needle is made of impact-resistant plastics material or pharmaceutically acceptable stainless steel.

4. Needle according to one or more of claims 1 to 3, **characterised in that** the needle is used to puncture a non-digestible plastics capsule in the powder inhaler.

5. Inhaler for the inhalation of powdered medicines from capsules, comprising:

   - a bottom part (1),
   - a plate (9) that can be locked to the bottom part (1) and by means of which the bottom part (1) can be closed, and a capsule holder (4) for receiving the capsules, which holder can be lowered into the bottom part (1),
   - a mouthpiece (12) that can be locked to the plate (9),

- a cap (13) that covers the mouthpiece (12) in a closed position and that is locked by means of a closure element (14), the bottom part (1), the plate, the mouthpiece (12) and the cap (13) being hingedly interconnected by means of a single joint, and
- an actuating member (7) that can be caused to move from a rest position and in the process interacts with at least one needle (6) that can be pushed into the capsule holder (4), **characterised in that** the needle (6) is formed according to any of claims 1 to 4.

6. Inhaler according to claim 5, **characterised in that** two triangular needles are used, which needles are formed as solid needles.

7. Inhaler according to claim 6, **characterised in that** the two triangular needles are arranged in parallel with one another in a head that can move against a spring.

8. Inhaler according to one or more of claims 5 to 7, **characterised in that** the needle or needles is/are used to puncture a capsule made of a non-digestible plastics material.

**Revendications**

1. Aiguille pour percer ou découper des capsules dans un inhalateur de poudre pour l'extraction de médicaments, de formulations médicamenteuses ou de mélanges de médicaments, **caractérisée en ce que** l'aiguille est une aiguille en matériau plein avec un affûtage sur trois arêtes ou un affûtage arrière, et l'aiguille possède un diamètre de 1,00 à 2,00 mm, dans laquelle l'aiguille présente un axe longitudinal central et une pointe d'aiguille qui est formée par l'affûtage sur trois arêtes ou l'affûtage arrière et qui se trouve sur l'axe longitudinal central.

2. Aiguille selon la revendication 1, **caractérisée en ce que** l'aiguille possède un affûtage ultra-long, long, moyen ou court.

3. Aiguille selon l'une des revendications 1 ou 2, **caractérisée en ce que** l'aiguille est constituée d'une matière plastique résistant aux chocs ou d'un acier inoxydable pharmaceutiquement inoffensif.

4. Aiguille selon une ou plusieurs des revendications 1 à 3, **caractérisée en ce que** l'aiguille sert à percer une capsule en matière plastique non digestible dans l'inhalateur de poudre.

5. Inhalateur pour l'inhalation de médicament sous forme pulvérulente à partir de capsules, présentant

- une partie inférieure (1)
- une plaque (9) pouvant être encliquetée avec la partie inférieure (1) avec laquelle la partie inférieure (1) peut être fermée, et un support de capsule (4) pouvant être escamoté dans la partie inférieure (1) pour la réception des capsules,
- un embout buccal (12) pouvant être encliqueté avec la plaque (9),
- un capuchon (13) qui couvre l'embout buccal (12) dans une position de fermeture et s'encliquette au moyen d'un élément de fermeture (14),
dans lequel la partie inférieure (1), la plaque, l'embout buccal (12) et le capuchon (13) sont reliés les uns aux autres de façon articulée par une seule articulation, et
- un organe d'actionnement (7) qui peut être déplacé d'une position de repos en un mouvement et coopère alors avec au moins une aiguille (6) pouvant être enfoncée dans le support de capsule (4),

**caractérisé en ce que**
l'aiguille (6) est réalisée selon l'une des revendications 1 à 4.

6. Inhalateur selon la revendication 5, **caractérisé en ce que** deux aiguilles à trois arêtes sont utilisées, lesquelles sont réalisées comme des aiguilles de matériau plein.

7. Inhalateur selon la revendication 6, **caractérisé en ce que** les deux aiguilles à trois arêtes sont disposées parallèlement l'une par rapport à l'autre dans une tête mobile contre un ressort.

8. Inhalateur selon une ou plusieurs des revendications 5 à 7, **caractérisé en ce que** l'aiguille ou les aiguilles sont

utilisées pour percer une capsule en une matière plastique non digestible.

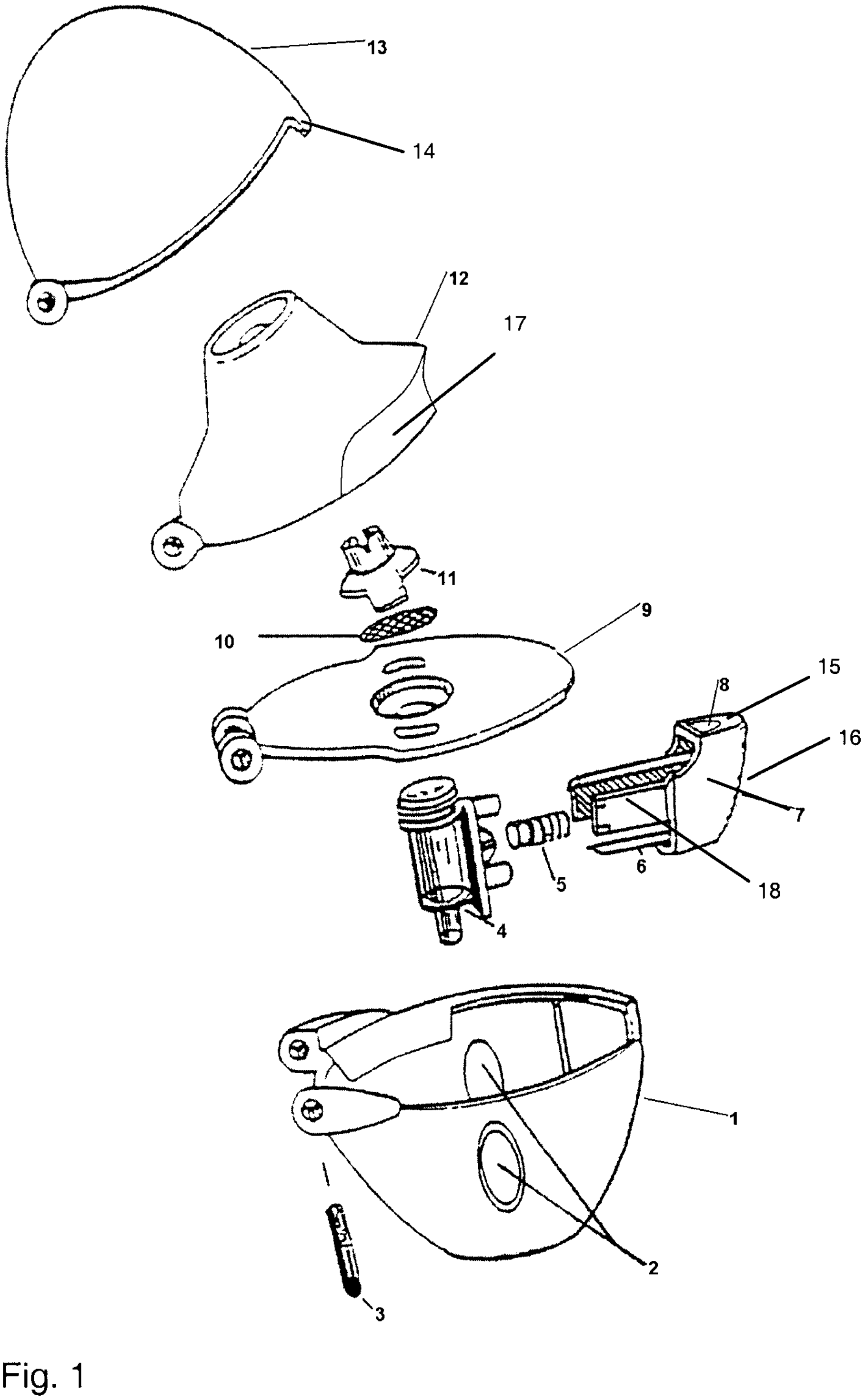


Fig. 1

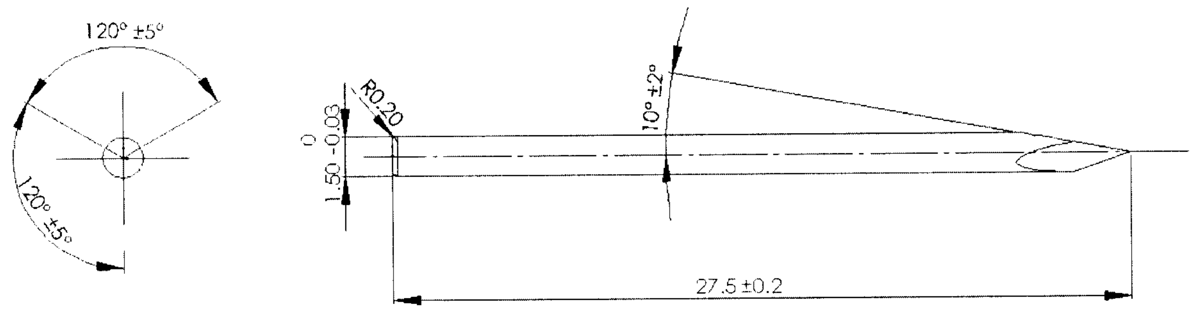


Fig. 2

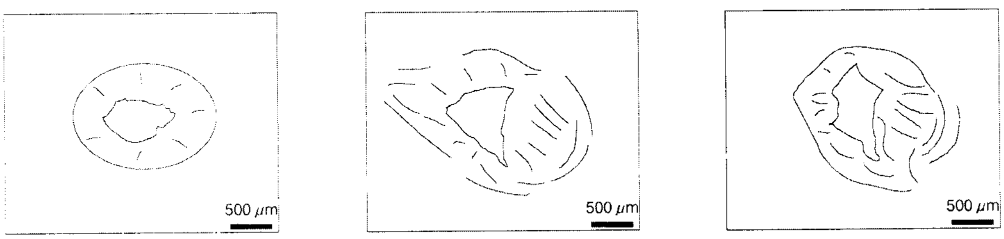


Bleistiftförmige Nadel Dreikantnadel Vierkantnadel

Fig. 3

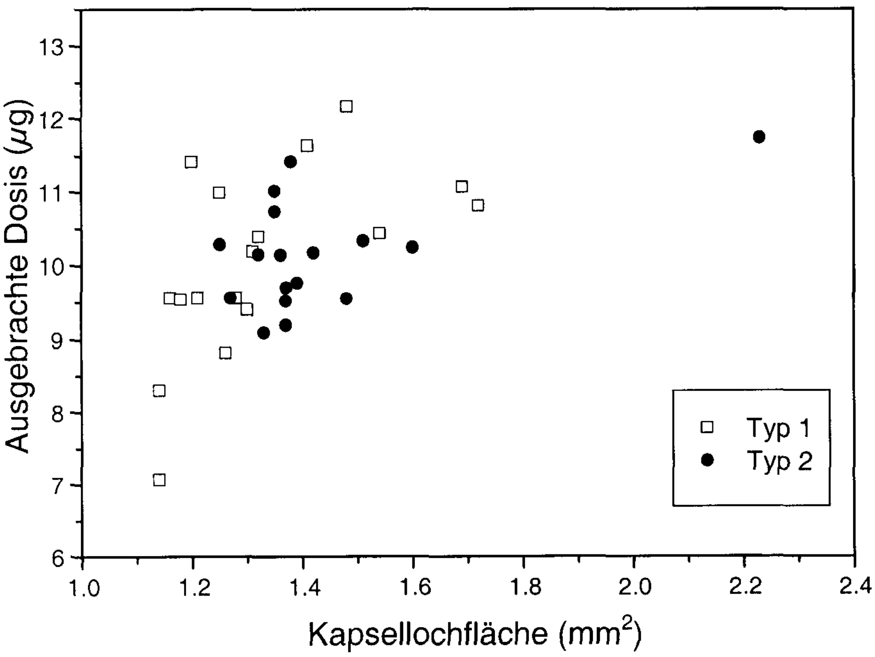
Ausgebrachte Dosis (μg)
13
12
11
10
9
8
7
6
1.0
1.2
1.4
1.6
1.8
2.0
2.2
2.4
Kapsellochfläche (mm²)
Typ 1
Typ 2

Fig. 4

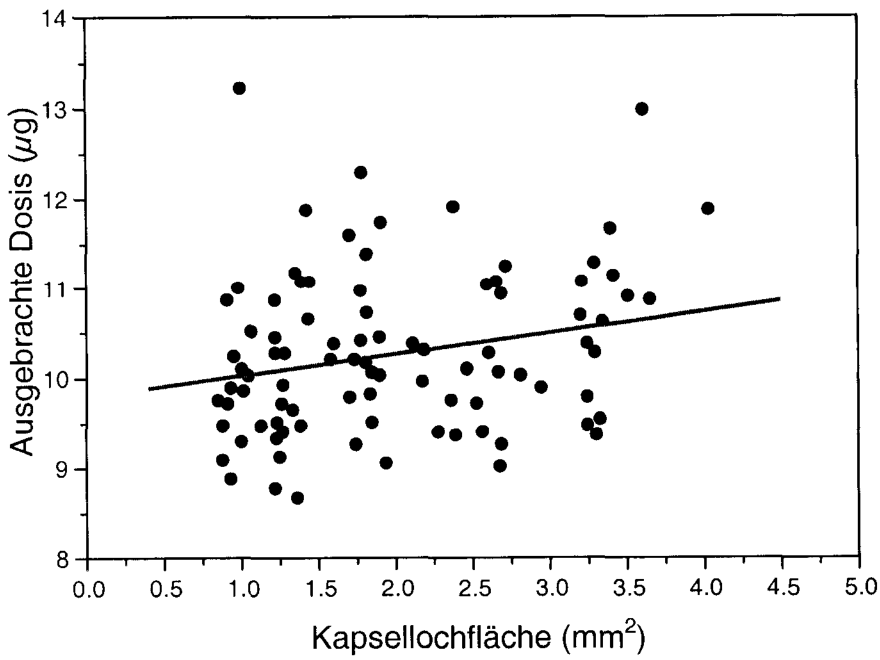
Ausgebrachte Dosis (μg)
14
13
12
11
10
9
8
0.0
0.5
1.0
1.5
2.0
2.5
3.0
3.5
4.0
4.5
5.0
Kapsellochfläche (mm²)

Fig. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0703800 B1
- EP 0911047 A1
- WO 2004082750 A1
- DE 3345722
- EP 0591136 A
- DE 4318455
- WO 9102558 A
- FR 2146202 A
- US 4069819 A
- EP 666085 A
- EP 869079 A
- US 3991761 A
- WO 9945987 A
- WO 200051672 A
- DE 202004005618
- EP 0460921 A
- GB 938828 A
- WO 0007572 A
- EP 1003478 A


### In der Beschreibung aufgeführte Nicht-Patentliteratur


- *Bell, J. Pharm. Sci.,* 1971, vol. 60, 1559
- *Cox, Brit. Med. J.,* 1969, vol. 2, 634
- Pharm. Eur. 2002, 554 ff